# EUROPEAN PATENT SPECIFICATION

(11) **EP 1 685 953 B1**
(45) Date of publication and mention of the grant of the patent: **19.03.2008**
(21) Application number: 05100475.2
(22) Date of filing: 26.01.2005
(51) Int. Cl.: B32B 5/26, B32B 5/28

(54) **Thermoplastic material, method for producing orthopaedic devices and orthopaedic devices produced therefrom**
Thermoplastisches Material, Verfahren zur Herstellung von orthopädischen Vorrichtungen und daraus hergestellte orthopädische Vorrichtungen
matiere thermoplastique, procede de fabrication des dispositifs orthopédiques et dispositifs orthopédiques ainsi fabriques

(43) Date of publication of application: 02.08.2006
(73) Proprietor: Runlite S.A., 4630 Micheroux (BE)
(72) Inventor: Hick, Christian, 4880 Aubel (BE); Smits, Jan F.A., 5709 MP Helmond (NL); Allard, Peter, 254 36 Helsingborg (SE); Dubois, Philippe Ghislain, 4260 Ciplet (Braives) (BE)
(74) Representative: Akerman, Marten Lennart

(56) References cited:
- US-B1- 6 186 966
- US-B1- 6 281 149

## Description

### Field of the invention

The present invention relates to a thermoplastic material and more particularly a fibre-containing substrate coated or impregnated with a thermoplastic composition. The material is suitable for producing for example orthopaedic devices. The invention also relates to a method for preparing such devices and devices produced with the method.

### State of the art

Fabrics of various types, such as woven, knitted and non-woven materials, and having a certain thickness are known per se within various fields. It is also known to produce orthopaedic devices using substrates impregnated with thermoplastic compositions.

US 4,238,522 discloses orthopaedic devices, materials and methods, and more particularly a bandage material manufactured from a substrate consisting of netting which is impregnated with a thermoplastic composition. The document fails to disclose how a substantial thickness of the material may be obtained, except by wrapping the material in several layers.

EP 0 934 749 discloses a supporting member manufactured by a plastics reinforced triaxially woven fabric. This material does not have a substantial thickness.

US 6,159,877 discloses a knit fabric material for orthopaedic support materials. The material is impregnated with a curable resin such that the prepared cast obtains a permanent shape that cannot later be altered.

US 2002/0,143,282 discloses an orthopaedic casting material comprising a double-knit material impregnated with hardening material. Once the cast has been hardened the shape cannot be altered.

US 2003/0,093,025 discloses an orthopaedic casting material comprising a double-knit material impregnated with hardening material. Once the cast has been hardened the shape cannot be altered.

US 5,284,031 discloses several knit ply fabrics with connecting layers. The document is only related to the fabric as such and is not concerned with producing composite materials wherein the fabric is impregnated with plastics.

US 6,281,149 is considered the most relevant prior art. The preamble of claim 1 is based on this document. The document discloses a multilayer laminated structure. The laminate comprises at least one three-dimensional woven material which preferably is combined with a stabiliser. A thermoplastic material is used as stabiliser, but the stabiliser is also activated and set (cured) by body temperature. The three-dimensional woven material may further be combined with a two-dimensional woven material, which is laminated to a thin thermoplastic film, such as polyethylene. This film has a thickness about 0.1 - 1.0 mils. The material of US 6,281,149 on one hand is combined with a thermoset material and, on the other hand, further is laminated with a thin thermoplastic film. The combinations disclosed do not result in a thermoplastic material as a whole.

In the art of for example orthopaedic devices, it is desired to obtain a material which is at the same time strong, lightweight and thermoplastic, and that is shapable when heated to a temperature which is tolerable for a human body. Preferably, the material should permit air circulation to improve the comfort further. None of the materials enumerated above achieves all the objectives at the same time.

### Summary of the invention

A material with the above characteristics is provided by the invention. The material comprises a substrate having two surface layers and a spacing layer placed therebetween. The substrate is coated or impregnated with a thermoplastic composition. Because of the spacing layer and the thermoplastic composition the material obtained is strong, has low weight and is shapable at suitable temperatures.

In a first aspect the invention provides a material comprising a fibre-containing substrate having two surface layers and an intermediate spacing layer with fibre strands connected between the surface layers; wherein the substrate is coated or impregnated with a thermoplastic composition.

Preferably, the spacing layer comprises strands running directly between the surface layers.

In a preferred embodiment, the substrate is a three-dimensional woven or knitted fabric.

In a second aspect the invention provides a method of producing an orthopaedic device, comprising the steps of: heating a piece of such a material until shapable; shaping the material into a desired shape in conformance with a body part, suitably directly on the body part; and allowing the piece of material to cool while maintaining the desired shape.

In a third aspect the invention provides an orthopaedic device thus produced.

### Brief description of the drawings

The invention will be described in detail below with reference to the accompanying drawings of which:
Fig 1 is a top view of a surface layer of a material of the invention;
Fig 2A is a cross-sectional view of material seen as indicated by the arrows IIa in Fig. 1; and
Fig 2B is a cross-sectional view of material seen as indicated by the arrows IIb in Fig. 1.

### Detailed description of preferred embodiments

The material according to the invention is suitable for orthopaedic devices, such as orthoses, braces, splints etc. The material has the strength necessary to achieve support and/or immobilisation. It is lightweight and permits circulation of air to be comfortable. The material comprises two main components, a substrate which is generally a fabric with a certain thickness and a thermoplastic composition making the substrate rigid when its temperature is below the softening point. The fibres of the substrate are arranged such that the material is reinforced to obtain the necessary strength.

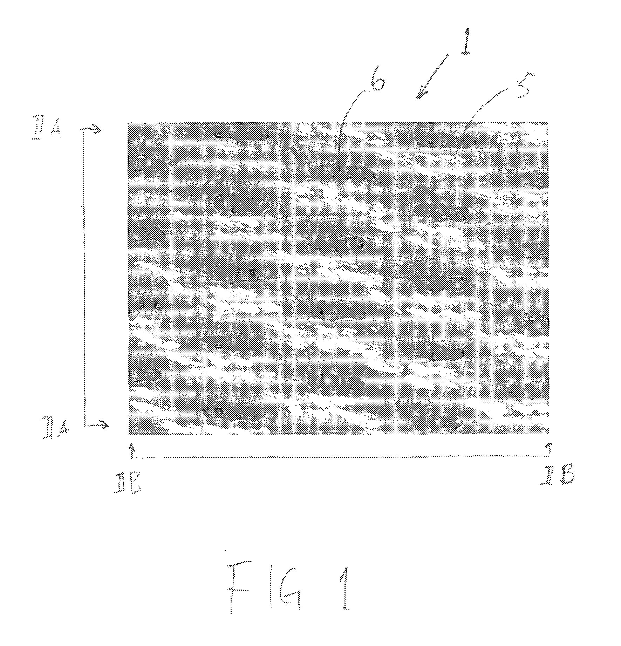

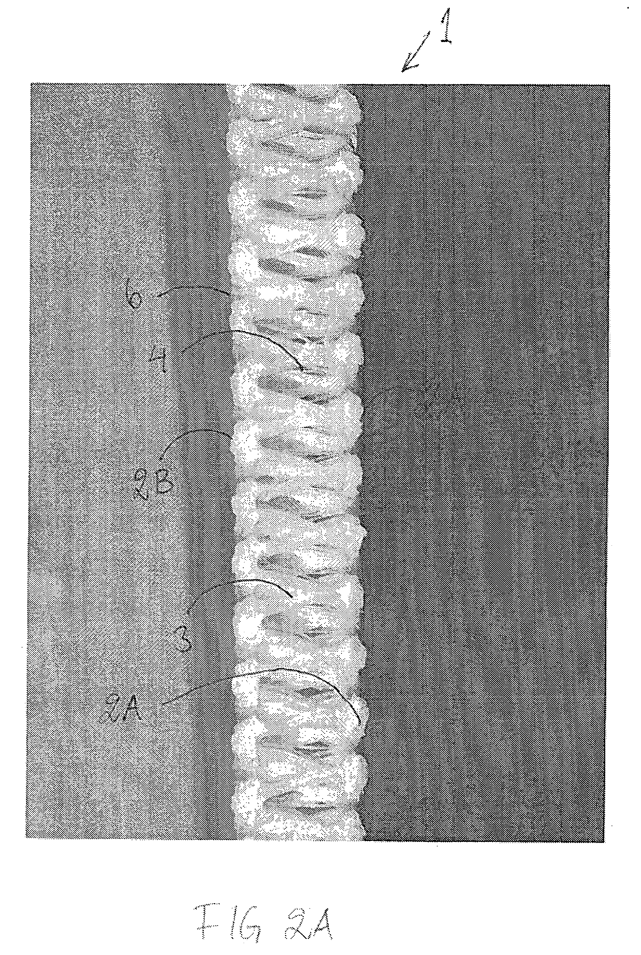

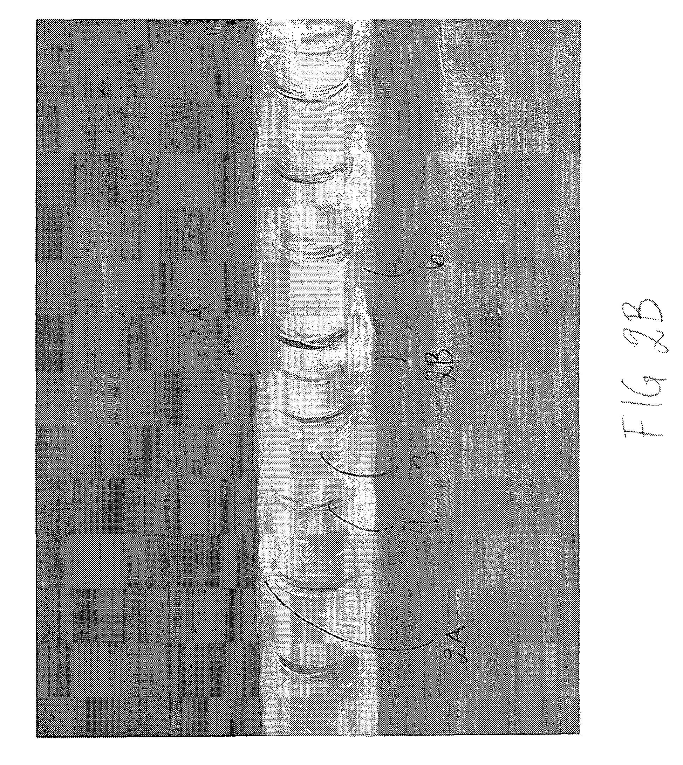

Figs 1, 2A and 2B illustrate an example of a substrate coated or impregnated with a thermoplastic composition. Even if the fibres of the substrate are coated with the composition the structure of the substrate is visible. The figures show the finished material ready for use.

The substrate of the material 1 in accordance with the invention is in the preferred embodiment a woven structure having surface layers 2A and 2B with openings 6 forming a netlike surface. The openings are holes through the whole material. Between the surface layers 2A and 2B there is a spacing layer 3. In the surface layers the fibres are woven together to form strand bundles 5 which are visible as ridges in the material between the wholes. In the spacing layer 3 the fibres are not woven together but are rather running directly between the surface layers 2A and 2B. At some locations some single connecting fibre strands 4 are visible. The substrate is preferably made from one long monofilament, both in the surface layers 2A and 2B and the spacing layer 3.

The connecting fibre strands 4 have shapes that depend on the weave structure. As seen in the plane of fig 2A, the strands 4 are running more or less straight between the surface layers, but with an angle to accommodate the openings 6. As shown in the plane of fig 2B, the strands 4 are more curved in this plane. When the material is softened the shape of the spacing layer strands 4 is also changed when shaping the whole material.

A suitable fabric is commercially available from the Danish company Tytex A/S under the trade name Spacer Fabric.

Other examples of fabrics are shown in US 5,284,031 showing knitted fabrics. The invention is also applicable with non-woven materials such as felt. The main feature of the invention is that there should be connecting fibre strands between the surface layers.

The substrate may be made from natural fibres such as cotton or wool, or synthetic fibres, such as nylon, polyester etc. Substrates made from polyester have proven to give excellent results. In the spacing layer 3 a certain rigidity of the fibres is advantageous. As a practical example only, the individual fibres may have a thickness of approximately 0.1 mm and the whole material may have thickness of about 5-10 mm (depending on the actual shape of the material).

The thermoplastic composition may be selected from a range of compositions. It should have the ability to be shaped at a temperature which is tolerable to the human body. For example a softening point at maximum 65-75°C is suitable. It should be capable of impregnating the substrate, in other words coating the strands of the substrate, the surface layers as well as the spacing layer. Suitable examples of compositions include polyols and the like. The preferred composition is polycaprolactone.

In use, the material may be shaped directly on the body part of a patient, usually with a stocking or the like closest to the skin. The material is heated above the softening point usually above 70°C, for example in a hot water bath or with a hot-air gun.

When the material is softened it is almost completely shapable to conform to the body part in question. By stretching the material the openings 6 will be contracted until eventually they are totally closed. The material may be stretched again in the perpendicular direction to open the openings 6 again. It is preferred that there remain some openings to permit air circulation through the material.

To complete the orthopaedic device the material may be provided with padding, straps and fasteners etc. as known in the art. Fasteners may be applied permanently to the material. The material may be shaped with fasteners already applied.

When the material is shaped to the desired form it is allowed to cool to regain its rigidity. Full strength is obtained gradually and well above normal body temperature. Then the device is fitted to the body part. If the fit is not perfect the first time the material may be heated again to adapt the shape. This shaping of the material may be repeated any number of times.

Thus, the present invention provides a material having several advantages over the prior art. The material has the strength necessary to achieve support and/or immobilisation when used in an orthopaedic device. At the same time, it is lightweight and permits circulation of air. The scope of the invention is only limited by the claims below.

## Claims

1. A material (1) comprising:
a fibre-containing substrate having two surface layers (2A, 2B) and an intermediate spacing layer (3) with fibre strands (4) connected between the surface layers (2A, 2B);
**characterised in that** the substrate is coated or impregnated with a thermoplastic composition, coating the strands of the substrate, the surface layers as well as the spacing layer.

2. A material according to claim 1, **characterised in that** the spacing layer (3) comprises individually coatable strands (4).

3. A material according to claim 1 or 2, **characterised in that** the spacing layer (3) comprises strands (4) running directly between the surface layers (2A, 2B).

4. A material according to claim 1, 2 or 3, **characterised in that** the strands (4) consist of a monofilament.

5. A material according to claim 1, 2, 3 or 4, **characterised in that** the surface layers (2A, 2B) comprise openings (6).

6. A material according to any one of claim 1, 2, 3, 4 or 5, **characterised in that** the substrate is a three-dimensional woven or knitted fabric.

7. A material according to any one of claim 1, 2, 3, 4 or 5, **characterised in that** the substrate is a three-dimensional non-woven material.

8. A material according to any one of the preceding claims, **characterised in that** the substrate is made of polyester.

9. A material according to any one of the preceding claims, **characterised in that** the thermoplastic composition is shapable at 65-75°C.

10. A material according to any one of the preceding claims, **characterised in that** the thermoplastic composition is a polyol.

11. A material according to claim 10, **characterised in that** the thermoplastic composition is polycaprolactone.

12. A method of producing an orthopaedic device, **characterised by** the steps of:
heating a piece of material (1) according to any one of claims 1 to 11 until shapable;
shaping the material into a desired shape in conformance with a body part, suitably directly on the body part; and
allowing the piece of material to cool while maintaining the desired shape.

13. A method according to claim 12, **characterised by** the step of attaching fasteners to the piece of material before or after shaping.

14. An orthopaedic device produced according to claims 12 or 13.

## Patentansprüche

1. Material (1), umfassend
ein faserhaltiges Substrat, das zwei Oberflächenschichten (2A, 2B) un d eine dazwischenliegende Abstandsschicht (3) mit Fasersträngen (4), die zwischenden Oberflächenschichten (2A, 2B) verbinden,hat
**dadurch gekennzeichnet, daß** das Substrat mit einer thermoplastischen Zusammensetzung beschichtet oder imprägniertis t , welchedi e Strängede s Substrats, die Oberflächenschichten wie auch die Abstandsschicht beschichtet.

2. Material nach Anspruch 1 , **dadurch gekennzeichnet, daß** die Abstandsschicht (3) individuellbeschichtbare Stränge (4) umfaßt.

3. Material nach Anspruch 1 oder 2, **dadurch gekennzeichnet, daß** die Abstandsschicht (3) Stränge (4) umfaßt , die direktzwischenden Oberflächenschichten(2A, 2B) verlaufen.

4. Material nach Anspruch 1, 2 oder 3, **dadurch gekennzeichnet, daß** die Stränge (4) aus einem Monofilamentbestehen.

5. Material nach Anspruch 1, 2, 3 oder 4, **dadurch gekennzeichnet, daß** die Oberflächenschichten (2A, 2B) Öffnungen (6) umfassen.

6. Material nach einem der Ansprüche1, 2, 3, 4 und 5, **dadurch gekennzeichnet, daß** das Substrat ein drei dimensionales gewebtes oder gestricktes Gewebeist.

7. Materialnacheinem der Ansprüche1, 2 , 3, 4 und 5, **dadurch gekennzeichnet, daß** das Substrat ein drei dimensionales Non-Woven-Materialist.

8. Material nach einem der vorangehenden Ansprüche, **dadurch gekennzeichnet, daß** das Substrat aus Polyesterbesteht.

9. Material nach einem der vorangehenden Ansprüche, **dadurch gekennzeichnet, daß** die thermoplastische zusammensetzung bei 65-75 °C formbar ist.

10. Material nach einem der vor angehenden Ansprüche, **dadurch gekennzeichnet, daß** die thermoplastische Zusammensetzung ein Polyolist.

11. Material nach Anspruch 10, **dadurch gekennzeichnet, daß** die thermoplastische Zusammensetzung Polycaprolacton ist.

12. Verfahren zur Herstellung einer orthopädischen Vorrichtung, **gekennzeichnet durch** die Schritte: Erwärmen eines Stücks von Material (1) nach einem der Ansprüche 1 bis 11, bis es formbar ist; Formen des Materials zu einer gewünschten Form in Anpassungan einen Körperteil, geeigneterweisedirektan dem Körperteilund Abkühlenlassendes Materialstück, währendes die gewünschte Form bei behält.

13. Verfahren nach Anspruch 12 , **gekennzeichnet durch** den Schritt einer Befestigung von Befestigungsmittelnan dem Materialstück vor oder nach dem Formen.

14. Orthopädische Vorrichtung, produziert nach Anspruch 12 oder13.

## Revendications

1. Matériau (1) comprenant:
un substrat contenant une fibre comportant deux couches de surface (2A, 2B) et une couche d'espacement intermédiaire (3) avec des brins de fibre (4) reliés entre les couches de surface (2A, 2B) ;
**caractérisé en ce que** le substrat est revêtu ou imprégné d'une composition thermoplastique, en revêtant les brins du substrat, les couches de surface de même que la couche d'espacement.

2. Matériau selon la revendication 1, **caractérisé en ce que** la couche d'espacement (3) comprend des brins pouvant être individuellement revêtus (4).

3. Matériau selon la revendication 1 ou 2, **caractérisé en ce que** la couche d'espacement (3) comprend des brins (4) courant directement entre les couches de surface (2A, 2B).

4. Matériau selon la revendication 1, 2 ou 3, **caractérisé en ce que** les brins (4) sont constitués d'un monofilament.

5. Matériau selon la revendication 1, 2, 3 ou 4, **caractérisé en ce que** les couches de surface (2A, 2B) comprennent des ouvertures (6).

6. Matériau selon l'une quelconque des revendications 1, 2, 3, 4 ou 5, **caractérisé en ce que** le substrat est un tissu tissé ou tricoté tridimensionnel.

7. Matériau selon l'une quelconque des revendications 1, 2, 3, 4 ou 5, **caractérisé en ce que** le substrat est un matériau non tissé tridimensionnel.

8. Matériau selon l'une quelconque des revendications précédentes, **caractérisé en ce que** le substrat est constitué de polyester.

9. Matériau selon l'une quelconque des revendications précédentes, **caractérisé en ce que** la composition thermoplastique peut être mise en forme de 65 à 75 °C.

10. Matériau selon l'une quelconque des revendications précédentes, **caractérisé en ce que** la composition thermoplastique est un polyol.

11. Matériau selon la revendication 10, **caractérisé en ce que** la composition thermoplastique est la polycaprolactone.

12. Procédé de production d'un dispositif orthopédique, **caractérisé par** les étapes consistant à :
chauffer une pièce de matériau (1) selon l'une quelconque des revendications 1 à 11 jusqu'à ce qu'elle puisse être mise en forme ;
mettre en forme le matériau à une forme souhaitée en conformité avec une partie du corps, directement sur la partie du corps de façon appropriée ; et
laisser la pièce de matériau refroidir tout en maintenant la forme souhaitée.

13. Procédé selon la revendication 12, **caractérisé par** l'étape consistant à fixer des attaches à la pièce de matériau avant ou après la mise en forme.

14. Dispositif orthopédique produit selon les revendications 12 ou 13.
